(19) Europäisches Patentamt
European Patent Office
Office européen des brevets

(11) Publication number: **0 271 575 B1**

(12) # EUROPEAN PATENT SPECIFICATION

(45) Date of publication of patent specification: **11.04.90**

(51) Int. Cl.$^5$: **B 01 J 21/16**

(21) Application number: **87905013.6**

(22) Date of filing: **11.06.87**

(86) International application number: **PCT/US87/01447**

(87) International publication number: **WO 88/00092 14.01.88 Gazette 88/02**

(54) **LAYERED METAL CHALCOGENIDES CONTAINING INTERLAYER CHALCOGENIDES AND THEIR SYNTHESIS.**

(30) Priority: **27.06.86 US 879787**

(43) Date of publication of application: **22.06.88 Bulletin 88/25**

(45) Publication of the grant of the patent: **11.04.90 Bulletin 90/15**

(84) Designated Contracting States: **BE DE FR GB IT NL SE**

(56) References cited:
**GB-A-1 528 982**
**US-A-3 677 971**
**US-A-4 065 380**
**US-A-4 110 251**
**US-A-4 515 901**
**US-A-4 629 713**
**US-A-4 637 991**

**IND. ENG. CHEM. PROD. RES. DEV., vol. 17, no. 3, September 1978, pages 214-219, American Chemical Society; P.G. BERCIK et al.: "Oligomerization of C3-C4 olefins using a novel nickel-aluminosilicate catalyst"**

(73) Proprietor: **MOBIL OIL CORPORATION**
**150 East 42nd Street**
**New York New York 10017 (US)**

(72) Inventor: **AUFDEMBRINK, Brent, Allen**
**5 Christopher Road**
**Voorhees, NJ 08043 (US)**
Inventor: **LANDIS, Michael, Eugene**
**26 North Horace Street**
**Woodbury, NJ 08096 (US)**

(74) Representative: **Colmer, Stephen Gary**
**Patent Department c/o Mobil Services Company Limited Mobil Court 3 Clements Inn**
**London WC2A 2EB (GB)**

(56) References cited:
**JOURNAL OF CHEMICAL SOCIETY, CHEMICAL COMMUNICATION, no. 2, 15th January 1987, pages 117-118; R. BURCH et al.: "Sulphide pillared interlayer clays"**

**EP 0 271 575 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

## Description

The present invention relates to layered metal chalcogenides containing interlayer chalcogenides as well as a method for preparing the same.

Many layered materials are known which have three-dimensional structures which exhibit their strongest chemical bonding in only two dimensions. In such materials, the stronger chemical bonds are formed in two-dimensional planes and a three-dimensional solid is formed by stacking such planes on top of each other, the interactions between the planes being weaker than the chemical bonds holding an individual plane together. The weaker bonds generally arise from interlayer attractions such as Van der Waals forces, electrostatic interactions, and hydrogen bonding. In those situations where the layered structure has electronically neutral sheets interacting with each other solely through Van der Waals forces, a high degree of lubricity is manifested as the planes slide across each other without encountering the energy barriers that arise with strong interlayer bonding. Graphite is an example of such a material. The silicate layers of a number of clay materials are held together by electrostatic attraction provided by ions located between the layers. In addition, hydrogen bonding interactions can occur directly between complementary sites on adjacent layers, or can be provided by interlamellar bridging molecules.

Laminated materials such as clays may be modified to increase their surface area. In particular, the distance between the layers can be increased substantially by absorption of various swelling agents such as water, ethylene glycol, amines and ketones, which enter the interlamellar space and push the layers apart. However, the interlamellar spaces of such layered materials tend to collapse when the molecules occupying the space are removed by, for example, exposing the clays to high temperatures. Accordingly, such layered materials having enhanced surface area are not suited for use in chemical processes involving even moderately severe conditions.

The extent of interlayer separation can be estimated by using standard techniques such as X-ray diffraction to determine the basal spacing, also known as "repeat distance" or "d-spacing". These values indicate the distance between, for example, the uppermost margin of one layer with the uppermost margin of its adjoining layer. If the layer thickness is known, the interlayer spacing can be determined by subtracting the layer thickness from the basal spacing.

Various approaches have been taken to provide layered materials of enhanced interlayer distance having thermal stability. Most techniques rely upon the introduction of an inorganic "pillaring" agent between the layers of a layered material. For example, U.S. Patent 4,216,188 discloses a clay which is crosslinked with metal hydroxide prepared from a highly dilute colloidal solution containing fully separated unit layers and a cross-linking agent comprising a colloidal metal hydroxide solution. However, this method requires a highly dilute forming solution of the clay (less than 1g/1) in order to effect full layer separation prior to incorporation of the pillaring species, as well as positively charged species of cross linking agents.

U.S. Patent 4,248,739 describes stable pillared interlayered clay prepared from smectite clays reacted with cationic metal complexes of metals such as aluminum and zirconium. The resulting products exhibit high interlayer separation and thermal stability.

U.S. Patent 4,176,090 discloses a clay composition interlayered with polymeric cationic hydroxy metal complexes of metals such as aluminum, zirconium and titanium. Interlayer distances of up to 16Angstrom are claimed although only distances restricted to about 9Angstrom are exemplified for calcined samples. These distances are essentially unvariable and related to the specific size of the hydroxy metal complex.

Silicon-containing materials are believed to be a highly desirable species of pillaring agent owing to their high thermal stability characteristics. U.S. Patent 4,367,163, describes a clay intercalated with silica prepared by impregnating a clay substrate with a silicon-containing reactant such as an ionic silicon complex, e.g., silicon acetylacetonate, or a neutral species such as $SiC1_4$. The clay may be swelled prior to or during silicon impregnation with a suitable polar solvent such as methylene chloride, acetone, benzaldehyde, tri- or tetraalkylammonium ions, or dimethylsulfoxide. This method, however, appears to provide only a monolayer of intercalated silica resulting in a product of small spacing between layers about 0.2-0.3 nm as determined by X-ray, diffraction.

In a first aspect, the present invention resides in a layered product comprising a layered chalcogenide of at least one element having an atomic number of 4, 5, 12 to 15, 20 to 33, 38 to 51, 56 to 83 and greater than 90, inclusive, and pillars of a chalcogenide of at least one element selected from Group IB, IIB, IIIA, IIIB, IVA, IVB, VA, VB, VIA, VIIA, VIIA of the Periodic Table of the Elements (Fisher Scientific Co. Cat. No. 5—702—10, 1978) separating the chalcogenide layers, at least one of the chalcogens of the layered chalcogenide and said pillars being other than oxygen.

Preferably, said pillars are formed of a polymeric chalcogenide and said product has a d-spacing of at least 2 nm.

For purposes of the present invention the term "chalcogenide" includes members of the group consisting of oxides, sulfides, selenides, tellurides, and polonides of elements other than those of Group VIB. For present purposes, polymeric chalcogenides are considered to include chalcogenides of two or more repeating units preferably three or more repeating units. The extent of polymerization of the interspathic polymeric chalcogenide is believed to affect the ultimate interlayer separation of the layered product.

It is also to be understood that as term "layered" chalcogenide or oxide is used herein in its commonly

accepted sense to refer to a material which comprises a plurality of separate chalcogenide or oxide layers which are capable of being displaced away from one another so that the spacing between adjacent layers is increased. Such displacement can be measured by x-ray diffraction techniques and/or by density measurement.

In a second aspect, the present invention relates to a method for preparing a layered product having adjacent layers separated by pillars of a chalcogenide of at least one element selected from Groups IB, IIB, IIIA, IIIB, IVA, IVB, VA, VB, VIA, VIIA, and VIIIA of the Periodic Table, which method comprises the steps of starting with a layered chalcogenide of at least one element having an atomic number of 4, 5, 12 to 15, 20 to 33, 38 to 51, 56 to 83 and greater than 90, inclusive, said layered chalcogenide having anionic sites associated therewith, physically separating the layers of the layered chalcogenide by introducing an organic cationic species between the layers at the anionic sites, introducing between the separated layers of the layered chalcogenide compound capable of conversion to a chalcogenide and converting said compound chalcogenide pillars separating adjacent layers of the layered chalcogenide, at least one of the chalcogens of the layered chalcogenide and the chalcogenide pillars being other than oxygen.

The method of the invention is particularly useful in that it permits the preparation of layered chalcogenide materials of relatively high interplanar distance (d-spacing), e.g., greater than 1 nm and preferably greater than 2 nm up to or even exceeding 3 nm. These materials are capable of being exposed to severe conditions such as those encountered in calcining, e.g., at temperatures of about 450°C for about two or more hours, e.g., four hours, in nitrogen or air, without significant decrease, say, e.g., less than about 10%, in interlayer distance. Furthermore, such layered chalcogenides can be prepared without the severe dilution often necessary to introduce the interspathic material in prior art techniques of interlayering. Finally, the size of interspathic chalcogenide contained within the final product can be greatly varied because the chalcogenide precursor species is introduced in an electrically neutral form such that the amount of interspathic material incorporated within the layered chalcogenide is not dependent upon the charge density of the original layered chalcogenide. Charge density should be taken into consideration in determining the suitability of the cationic species introduced between the layers in the procedure used to prop open the layers prior to pillaring.

Preferably, the other chalcogen of the layered chalcogenide and the chalcogenide pillars is oxygen.

The method of the present invention utilizes a layered chalcogenide starting material which contains anionic sites having interlayer cations associated therewith. Such cations may include hydrogen ion, hydronium ion and alkali metal cation. The starting material is treated with a "propping" agent comprising a source of organic cation, such as an organoammonium cation in order to effect an exchange of or addition to the interlayer cations in the starting material resulting in the layers of the starting material being propped apart. The source of organic cation in those instances where the interlayer cations include hydrogen or hydronium ions may include a neutral compound such as organic amine which is converted to a cationic analogue during the "propping" treatment. In some instances, it may be desirable to remove excess propping agent which is not electrostatically bound within the layered starting material in order to permit the subsequent addition of greater amounts of chalcogenide precursor. Such removal may be effected by washing out the propping agent with a suitable solvent.

The foregoing treatment results in the formation of a layered metal chalcogenide of enhanced interlayer separation depending upon the size of the organic cation introduced. In one embodiment, a series of organic cation exchanges is carried out. For example, an organic cation may be exchanged with an organic cation of greater size, thus increasing the interlayer separation in a step-wise fashion. Preferably contact of the layered oxide with the propping agent is conducted in an aqueous medium so that water is trapped between the layers of the "propped" chalcogenide.

After the ion exchange, the organic-"propped," species is treated with a compound capable of conversion, preferably by hydrolysis, to chalcogenide pillars separating the substrate layers. Where the conversion involves hydrolysis, this may be carried out using water already present in organic-"propped" layered chalcogenide material. In this case, the extent of hydrolysis may be modified by varying the extent to which the organic-"propped" species is dried prior to addition of the polymeric chalcogenide precursor.

It is preferred that the organic cation deposited between the layers is capable of being removed from the layered chalcogenide material without substantial disturbance or removal of the interspathic chalcogenide or its precursor. For example, organic cations such as n-octylammonium may be removed by exposure to elevated temperatures, e.g., calcination, in nitrogen or air or chemical oxidation conditions, preferably after the interspathic chalcogenide precursor has been converted to the chalcogenide pillars.

The products of the present invention, especially when calcined, exhibit high surface area, e.g., greater than 200, 400 or even 600 $m^2/g$, and thermal and hydrothermal stability making them highly useful as catalysts or catalytic supports, for hydrocarbon conversion processes for example, cracking and hydrocracking.

The layered chalcogenides used in the invention are layered chalcogenides of elements having an atomic number of 4, 5, 12 to 15, 20 to 33, 38 to 51, 56 to 83 and greater than 90. Where the chalcogen of the pillars is not oxygen, the layered chalcogenide is preferably an oxide. Suitable oxides are oxides of aluminum and silicon, such as clays, e.g. as bentonite. Preferably, however, the layered chalcogenide is "non-swellable" which is intended to distinguish from conventional clays which contain octahedrally coordinated metal oxide sheets bonded to tetrahedrally coordinated silica sheets and which undergo

substantial swelling, sometimes by an essentially unbounded amount, when contacted with water. As used herein in relation to a layered chalcogenide material, the term "non-swellable" is defined as meaning a layered chalcogenide material, which, when contacted with at least 10 grams of water per gram of the layered chalcogenide at 23°C for 24 hours, exhibits an increase in d-spacing no greater than 5 Angstrom as compared with the material before treatment. Included among these materials are $H_2Ti_3O_7$, $Na_2Ti_3O_7$ and $KTiNbO_5$ as well as certain layered silicates, for example, magadiite, natrosilite, kenyaite, makatite, nekoite, kanemite, okenite, dehayelite, macdonaldite and rhodesite which, unlike swellable clays, lack octahedral sheets, i.e., sheets composed of atoms which are octahedrally coordinated with oxygen atoms. In some cases it has been found preferable that these layered silicates are treated by contacting with one or more polar organic solvents or water prior to or during exchange with the source of organic cation. The polar organic solvent used should exhibit electric dipole moments in the gas phase of at least 3.0 Debyes (D), preferably at least 3.5, and most preferably at least about 3.8D. Examples of suitable organic solvents are dimethylsulfoxide (DMSO) and dimethylformamide (DMF). A table of selected organic compounds and their electric dipole moments can be found in CRC Handbook of Chemistry and Physics, 61st Edition, 1980-1981 at pages E-64 to E-66.

In one preferred embodiment, the starting material is a layered chalcogenide, preferably oxide, of a Group IV A metal such as titanium, zirconium and hafnium, with a layered titanate, e.g., a trititanate such as $Na_2Ti_3O_7$, being particularly preferred. Trititanates are commercially available materials whose structure consists of anionic sheets of titanium octahedra with interlayer alkali metal cations which can be exchanged for interspathic H+ and $H_3O$+ ions. A method for making such material may be found in U.S. Patent 2,496,993. It is known that the interlayer distance of $Na_2Ti_3O_7$ may be increased by replacing interlayer sodium ions with larger octylammonium ions. (See, Weiss *et al.,* Angew. Chem/72 Jahrg. 1960/ Nr/2, pp 413-415-) However, the organic-containing trititanate is highly susceptible to heat which can remove the organic material and cause collapse of the layered structure. The present invention serves to introduce a stable polymeric chalcogenide, preferably comprising an element selected from the group consisting of silicon, germanium, tin and lead, e.g., polymeric silica, between adjoining layers resulting in a heat-stable material which substantially retains its interlayer distance upon calcination percent.

In another preferred embodiment, the chalcogenide starting material is a layered silicate lacking octahedral sheets, either in natural or synthetic form, such as magadiite, kenyaite or makatite, which may contain elements other than silicon capable of tetrahedral coordination in its framework, e.g., Al, B, Co, Cr, Fe, Ga, In, Ni and Zr. Such layered silicates can be prepared by co-crystallizing in the presence of the required non-silicon tetravalent elements, or alternatively, non-silicon framework elements already in the layered silicate may be substituted by a tetracoordinate element. For example, kenyaite containing boron in its framework when treated with aluminum nitrate results in a kenyaite which contains aluminum in its framework.

Another embodiment of the present invention resides in preparing synthetic magadiite-type materials which contain pillars formed of non-oxide polymeric chalcogenides. Synthetic magadiite is readily synthesized hydrothermally from an aqueous reaction mixture containing inexpensive sources of silica and an alkali metal hydroxide caustic. Tetracoordinate elements other than silicon, e.g., those selected from the group consisting of Al, B, Cr, Fe, Ga, Co, In, Ni and Zr, may be added to the reaction mixture to produce synthetic magadiite-type layered silicates. Preferably, such elements are selected from the group consisting of Al and Fe. An organic directing agent may also be added to the reaction mixture. The reaction mixture for synthetic magadiite-type materials can be described in molar ratios as follows:

$SiO_2/X_2O_3$ = 10 to infinity where X can be Al, B, Cr, Co, Fe, Ga, and/or Ni

M+OH-/$SiO_2$ = 0 to 0.6, (preferably 0.1-0.6) M = any alkali metal

$H_2O/SiO_2$ = 8 - 500

$R/SiO_2$=0-0.4

where R can be an organic such as benzyltriethylammonium chloride, benzyltrimethyl- ammonium chloride, dibenzyldimethylammonium chloride, N,N'-dimethylpiperazine, triethylamine, or other quaternary compounds or heterocyclic amines.

The reaction mixture can be maintained at a temperature of 100 to 200°C for anywhere from 1 to 150 days in order to form a product having the following composition:

% N = 0 - 3, e.g., 0 to 0.3

$SiO_2/X_2O_3$ = 10 to infinity where X may be in the tetrahedral or octahedral position

$M_2O/SiO_2$ = 0 to 0.5, e.g., 0.05 - 0.1

The synthetic layered silicate materials thus prepared are of low surface area. Introduction of pillars of

a non-oxide chalcogenide can increase the surface area of these materials. Another embodiment aspect of the present invention resides in preparing synthetic kenyaite-type materials which contain pillars of non-oxide polymeric chalcogenides. Kenyaite, a layered silicic acid which is known to exist in nature as a sodium salt $Na_2$ $Si_{22}O_{45}H_2O$ can be prepared in the potassium form $K_2Si_{22}O_{45}$ $10H_2O$ in the laboratory. Synthetic kenyaite is readily synthesized hydrothermally from a reaction mixture containing inexpensive sources of silica and caustic, preferably KOH. Again Tetracoordinate elements other than silicon, especially B, Al and Zr may be added to the reaction mixture to produce synthetic kenyaite-type layered silicates. $Al(NO_3)_3.9H_2O$ and aluminum-tri-sec-butoxide are suitable reagents for the introduction of non-silicon tetracoordinate elements in the kenyaite framework.

In another embodiment of the present invention, high surface area porous molecular sieve materials are prepared from layered transition metal non-oxide chalcogenides. These are of particular interest in that they may contain transition metal atoms having partially filled d-orbitals, which may result in metal-to-metal bonding within the layers and hence unusual catalytic chemistry.

Examples of layered transition metal chalcogenides are well-known in the art. The structure of these materials and their intercalation with alkali and alkaline earth metal ions are taught in Schollhorn, R.; Meyer, H., *Mat. Res. Bull.* 1974, *9*, 1237 and Jacobson A. J., "Intercalation Chemistry" Academic Press, N.Y., 1982, P229. Organoammonium ions can also occupy the interlayer regions of these materials as taught in Schollhorn R., Zagefka H., Butz T., Lerf A., *Mat. Res. Bull.* 1979, *14*, 369; Schollhorn R., Sick E., Lerf A. *Mat. Res. Bull.* 1975, *10*, 1005; Weiss A. and Ruthardt R., *Z. Natur. Forsch.* 1973, *286*, 249 and Schollhorn R., Sick E., and Weiss A, Z *Natur. Forsch.*, 1973 286, 168.

Suitable layered transition metal chalcogenides have the empirical formula $Mx_2$ where X is selected from the group consisting of S, Se and Te and M is a transition metal selected from Groups IVA, IVB, VA, VIA, VIIA and VIIIA of the Periodic Chart. Preferably M is selected from the group consisting of Ti, Zr, Hf (Group IVA); Sn, Pb (Group IVB); V, Nb, Ta (Group VA); Mo, W (Group VIA); Tc, Re (Group VIIA) and Pt (Group VIIIA). M may be in octahedral coordination and/or trigonal prismatic coordination with the X atoms. X is preferably S so that $TiS_2$ is a layered material particularly well-suited to the present invention.

These layered metal dichalcogenide materials are generally electrically neutral. However, they can be reduced, for example, by contact with an aqueous alkali metal salt which acts as a reducing agent, e.g. $Na_2S_2O_4$ in the case of $MS_2$ materials.Other reducing agents can include alkali metal salts of borohydride or sulfide. The reduction of the layered material results in the formation of a negative charge on each layer which becomes balanced by the presence of the alkali metal ion between the layers. Subsequent treatment with propping agents, particularly if they are cationic, can be significantly enhanced by this reduction treatment. However, significant absorption of a neutral swelling agent can occur with $MX_2$ materials even in the absence of such reduction. Indeed, the amount of swelling agent, e.g. n-alkylamine which is incorporated into the layered material can be dependent on the nature of the metal atom in the layer, i.e., the electronic band structure of the layered material. This can also affect the amount of polymeric chalcogenide precursor, e.g. polymeric oxide precursor such as tetraethylorthosilicate which may later be sorbed between the layers.

Layered metal dichalcogenides $MX_2$ pillared by the method of the present invention, particularly those where X is sulfur are believed suitable for use in petroleum processing, particularly in the removal of heteroatoms from resid chargestocks.

Another layered oxide material suitable for use in the present invention is a perovskite-related layered oxide. perovskite-related layered oxides are known in the art and are described, for example by Dion M, Ganne M., Tournoux M, in *Mat. Res. Bull*, 1981, *16*- 1429; Galasso F., "Structure, Properties and Preparation of Perovskite Type Compounds", Pergamon Press, 1969; and Jacobson et al, *Inorg. Chem.*, 1985, *24*, 3727. These materials as well as their organic-swelled analogues, e.g., those which are octylamine-swelled, are disclosed in U.S. Patent No. 4,593,013. Such materials can be treated by the method of the present invention to incorporate pillars of non-oxide chalcogenides therein.

The perovskite-related layered-oxides used herein may be represented by the formula $M_m[A_{n-1}B_nO_{3n+1}]$, although oxygen-deficient variants of this formula are known and may also be employed. In this formula M is a charge-balancing interspathic cation; $[A_{n-1}B_nO_{3n+1}]$ represents a perovskite-like layer, wherein A is one more metal atoms capable of occupying 12-coordinate sites and B is a metal atom capable of occupying 6-coordinate sites, m is greater than 0, preferably less than or equal to 1; and n is greater than or equal to 2, preferably 3 to 7. Each layer comprises a cubic arrangement of corner-shared $B0_6$ octahedra with A occupying a 12-coordinated site in the center of each cube. For purposes of the present invention, the term "cubic arrangement" can include any generally cubic or pseudo-cubic arrangement.

The thickness of each layer in terms of $BO_6$ octahedra is denoted by n. In other words, the layers can vary, for example, between 3 and 7 $BO_6$ octahedra in thickness, depending on the perovskite-like layered material. Perovskite-like layered materials treated by the method of the present invention preferably have layers of a low charge density in order to exhibit the ion exchange properties necessary for incorporation of the more common propping agents prior to intercalation with polymeric chalcogenide precursor. Although some perovskite-like layered materials have a charge density per formula unit of two or more, the perovskite-like layered materials treated by the present invention preferably have a charge density of one or less. However, it is possible that a propping agent of requisite shape and charge can exchange with the interspathic cations in materials where m is greater than 1.

M in the above perovskite formula can be a monovalent, divalent or trivalent cation, preferably a monovalent cation selected from the group consisting of Li, Na, K, Rb, Cs, $NH_4$ and H, while A can be one or more mono-, di- or trivalent cations selected from the group consisting of Groups IA, IIA and IIIB and the lanthanides and B can be one or more transition metals selected from Re and Groups IVB, VB and VIB. In one preferred embodiment, $A_{n-1}$ can be $Ca_2Na_{n-3}$ and B is Nb; in other words, the perovskite layer is represented by the formula $Ca_2Na_{n-3}Nb_nO_{3n+1}$. Preferably in such cases, M is K and n is 3, e.g., $KCa_2Nb_3O_{10}$.

During preparation of a pillared perovskite-related layered oxide according to the present invention it has been found beneficial to carry out the swelling step utilizing a cationic species or cationic species precursor at temperatures above ambient, say, e.g. 70 to 110°C, say about 100°C. Similarly, the interspathic non-oxide polymeric chalcogenide precursor is preferably introduced into the layered oxide at temperatures above ambient, e.g. 70 to 100°C, say about 80 to 90°C.

Further suitable layered oxides are layered metal oxides in which each layer has the general formula $[M_x\square_yZ_{2-(x+y)}O_4]\alpha$- wherein M is at least one metal of valence n where n is an integer between 0 and 7, $\square$ represents a vacancy site, Z is a tetravalent metal, preferably titanium, and wherein

$\alpha = 4y - x(n - 4)$ and preferably is 0.6 - 0.9, and

$0 < x + y < 2$

Interposed between the layers of the oxide will be charge-balancing cations A of charge m wherein m is an integer between 1 and 3, preferably 1. Preferably A is a large alkali metal cation selected from the group consisting of Cs, Rb and K and M is at least one divalent or trivalent metal cation selected from the group consisting of Mg, Sc, Mn, Fe, Cr, Ni, Cu, Zn, In, Ga and Al. For example, M can be both In and Ga. Structurally, these mixed metal oxides consist of layers of $(M_x\square_yZ_{1-x-y})O_6)$ octahedra which are *trans* edge-shared in one dimension and *cis* edge-shared in a second dimension forming double octahedral layers which are separated by the A cations in the third dimension. Titanometallate materials of this formula can be prepared by high temperature fusion of a mixture of 1) metal (M) oxide source, 2) alkali metal carbonate or nitrate and 3) titanium dioxide; or by fusion of a mixture of alkali metallate and titanium dioxide. Such fusion can be carried out in our air in ceramic crucibles at temperatures ranging between 600 to 1100°C, after the reagents have been thoroughly ground to an homogeneous mixture. The resulting product is ground to 20 to 250 mesh (0.84 to 0.063 mm), preferably about 100 mesh (0.149 mm), prior to the organic swelling and polymeric chalcogenide intercalation steps. The ground titanometallate layered material is then treated with a "propping agent" as described earlier, for example aqueous alkylammonium halide, say, octylammonium chloride. It has been found necessary to maintain a low hydrogen ion concentration to prevent decomposition of the titanometallate structure as well as to prevent preferential sorption of hydrogen ion over the propping agent. A pH range of 6 to 10, preferably 7 to 8.5 is generally employed during treatment with the propping agent. After this treatment, it has been found advantageous to wash out excess propping agent using a suitable solvent followed by washing with water prior to treatment with the chalcogenide precursor. Such washing permits greater incorporation of the chalcogenide precursor by the layered titanometallate while the water treatment allows penetration of water into the interlayer treatment which assists in hydrolyzing the precursor.

Further description of the layered titanometallate starting materials and their methods of preparation can be found in the following references:

Reid, A. F.; Mumme, W. G.; Wadsley, A. D. *Acta Cryst.* (1968), *B24*, 1228; Groult, D.; Mercy, C.; Raveau, B. *J. Solid State Chem.* 1980, *32* 289; England, W. A.; Burkett, J. E.; Goodenough; J. B., Wiseman, P. J.*J. Solid State Chem.* 1983, *49* 300.

Use of the above layered metal oxides as the layered metal chalogenide of the present invention permits inclusion of different metal atoms into the layered chalcogenide material being treated which allows potential catalytically active sites to be incorporated in the stable chalcogenide layer itself. Moreover, variable amounts of metal atoms may be added to provide a catalyst with optimum activity for a particular process. Furthermore, the infinite trans-edge shared layer structure of the titanometallates instead of the sheared 3-block structure of $Na_2Ti_3O_7$ may reduce or eliminate shearing of the layers as a possible mechanism for thermal or hydrothermal decomposition of the calcined intercalated material. These titanometallate materials may possess even greater thermal stability than silicotitanate molecular sieves. In addition, the variable charge density on the oxide layer possible for these layered metal oxide materials, due to the various oxidation states of the incorporated metal atom and their varying stoichiometry, may allow variation in the amount of the organic cationic species which can be exchanged into the material. This in turn, permit variation of the ultimate concentration of the chalcogenide pillars between the layers of the final product.

According to the method of the invention, the layered chalcogenide material is treated with an organic compound capable of forming cationic species such as organophosphonium or organoammonium ion, before adding the polymeric chalcogenide source. Insertion of the organic cation between the adjoining layers serves to physically separate the layers in such a way as to make the layered chalcogenide receptive to the interlayer addition of an electrically neutral, hydrolyzable, chalcogenide precursor. In particular, alkylammonium cations have been found useful in the present invention. Thus $C_3$ and larger alkylammonium, e.g., n-octylammonium, cations are readily incorporated within the interlayer spaces of the layered chalcogenides, serving to prop open the layers in such a way as to allow incorporation of the chalcogenide precursor. The extent of the interlayer spacing can be controlled by the size of the

organoammonium ion employed so that use of the n-propylammonium cation can achieve an interlaver spacing of 0.2-0.5 nm, whereas to achieve an interlayer opening of 1 to 2 nm an n-octylammonium cation or a cation of equivalent length is required. Indeed, the size and shape of the organic cation can affect whether or not it can be incorporated within the layered chalcogenide structure at all. For example, bulky cations such as tetrapropylammonium are generally undesirable for use in the present method while n-alkyl ammonium cations, such as those derived from primary n-alkylamines and $R_3R^1N^+$ cations, where R is methyl or ethyl and $R^1$ is an n-alkyl group with at least 5 carbon atoms, are preferred. The organic ammonium cations separating the chalcogenide layers may also be formed in situ by reaction of a neutral amine species with interlayer hydrogen or hydronium cations of the layered chalcogenide starting material. Alternatively, where the interlayer cations of the layered chalcogenide starting material are alkali metal cations, the organic ammonium cation may be formed by initially combining an amine and an aqueous acid solution, such as hydrochloric acid, and then treating the layered chalcogenide with the resulting aqueous organoammonium ion solution. In either case, the treatment is preferably conducted in aqueous media so that water is then available to hydrolyze the electrically neutral, hydrolyzable chalcogenide precursor subsequently introduced into the "propped" product.

The interspathic chalcogenide pillars formed between the layers of the chalcogenide starting material may include a chalcogenide, preferably oxide or sulfide, of zirconium or titanium or more preferably of an element selected from Group IVB of the Periodic Table (Fischer Scientific Company Cat. No. 5-702-10, 1978), other than carbon, i.e., silicon, germanium, tin and lead. Other such elements may include those of Group VA, e.g., V, Nb, and Ta, those of Group IIA, e.g., Mg or those of Group IIIB, e.g., B. Most preferably, the pillars include polymeric silica when the layered starting material is a non-oxide chalcogenide, and include titanium disulfide when the layered starting material is an oxide. In addition, the chalcogenide pillars may include an element which provides catalytically active acid sites in the pillars, preferably aluminum.

The chalcogenide pillars are formed from a precursor materia1 which is preferably introduced between the layers of the organic "propped" species as a cationic, or more preferably, electrically neutral, hydrolyzable compound of the desired elements, e.g., those of Group IVB. The precursor material is preferably an organometallic compound which is a liquid under ambient conditions. In particular, hydrolyzable. compounds, e.g., alkoxides, of the desired elements of the pillars are utilized as the precursors. Suitable polymeric silica precursor materials include tetraalkylsilicates, e.g., tetrapropylorthosilicate, tetramethylorthosilicate and, most preferably, tetraethylorthosilicate. Where the pillars are also required to include alumina, a hydrolyzable aluminum compound can be contacted with the organic "propped" species before, after or simultaneously with the contacting of the layered chalcogenide with the silicon compound. Preferably, the hydrolyzable aluminum compound employed is an aluminum alkoxide, e.g., aluminum isopropoxide. If the pillars are to include titania, a hydrolyzable titanium compound such as titanium alkoxide, e.g., titanium isopropoxide, may be used.

In addition, the chalcogenide precursor may contain zeolite precursors such that exposure to conversion conditions results in the formation of interspathic zeolite material as at least some of the chalcogenide pillars. In this case, the source of organic cation propping agent exchanged with the interspathic cations may act as a zeolite synthesis directing agent. Suitable sources interspathic organic cation may include primary monoalkylamines or primary monoalkylammonium ions such as n-octylamine or n-octylammonium ion. U.S. Patent No. 4,151,189 discloses reagents and conditions suitable in forming the zeolite component of this embodiment. The patent discloses oxides of aluminum, silicon and alkali metal suitable for zeolite synthesis as well as zeolite synthesis directing agents such as sources of organic nitrogen cation, like $C_4$-$C_{10}$ n-alkylamines. Suitable sources of alumina include sodium aluminate, aluminum sulfate and alumina while suitable sources of alkali metal include alkali metal hydroxide such as sodium hydroxide. Suitable reaction conditions include heating the layered material containing the zeolite precursors to a temperature of from 99°C to 260°C for a period from 6 hours to 60 days, preferably 149°C to 232°C for a period from 12 hours to 8 days.

After hydrolysis to produce the chalcogenide pillars and calcination to remove the organic propping agent, the final pillared product may contain residual exchangeable cations. Such residual cations in the layered material can be ion exchanged by known method with other cationic species to provide or alter the catalytic activity of the pillared product. Suitable replacement cations include cesium, cerium, cobalt, nickel, copper, zinc, manganese, platinum, lanthanum, aluminum, ammonium, hydronium and mixtures thereof.

When used as a catalyst, it may be desirable to incorporate the pillared product of the invention with another material, i.e. a matrix, resistant to the temperatures and other conditions employed in organic conversion processes. Such materials include active and inactive materials and synthetic or naturally occurring zeolites as well as inorganic materials such as clays, silica and/or metal oxides. The latter may be either naturally occurring or in the form of gelatinous precipitates or gels including mixtures of silica and metal oxides. Use of a matrix material in conjunction with the pillared product, i.e. combined therewith, which is active, tends to improve the conversion and/or selectivity of the catalyst in certain organic conversion processes. Inactive materials suitably serve as diluents to control the amount of conversion in a given process so that products can be obtained economically without employing other means for controlling the rate of reaction. These materials may be incorporated into naturally occurring clays, e.g.

bentonite and kaolin, to improve the crush strength of the catalyst under commercial operating conditions. Said materials, i.e. clays, oxides, etc., functions as binders for the catalyst. It is desirable to provide a catalyst having good crush strength because in commercial use it is desirable to prevent the catalyst from breaking down into powder-like materials. These clay binders have been employed normally only for the purpose of improving the crush strength of catalyst.

Naturally occurring clays which can be composited with the pillared product include the montmorillonite and kaolin families which include the subbentonites, and the kaolins commonly known as Dixie, McNamee, Georgia and Florida clays or others in which the main mineral constituent is halloysite, kaolinite, dickite, nacrite, or anauxite. Such clays can be used in the raw state as originally mined or initially subjected to calcination, acid treatment or chemical modification. Matrix materials useful for compositing with the pillared product also include inorganic oxides, notably alumina or silica.

In addition to the foregoing materials, the pillared product of the invention can be composited with a porous matrix material such as aluminum phosphate, silica-alumina, silica-magnesia, silica-zirconia, silica-thoria, silica-beryllia, silica-titania as well as ternary compositions such as silica-alumina-thoria, silica-alumina-zirconia, silica-alumina-magnesia and silica-magnesia-zirconia. The relative proportions of finely divided pillared product and inorganic oxide gel matrix vary widely, with crystal content of the pillard product ranging from 1 to 90 percent by weight and more usually, particularly when the composite is prepared in the form of beads or extrudates, in the range of 2 to 80 weight percent of the composite.

The invention will now be described with reference to the following examples.

Example 1

a) A.gel was produced by mixing 400 g Cabosil silica in 54.4 g 98% NaOH and 1.4 kg water. The gel was crystallized in a 2 liter polypropylene jar at 100°C for 23 days to produce synthetic magadiite, which was then filtered, washed with hot water and dried at 121°C (250°F) overnight. The dried product had the following composition (wt%):

| | |
|---|---|
| $Si0_2$ | 83.3 |
| $Na_20$ | 6.9 |
| $A1_20_3$ | 0.01 |

100g of the dried product were added to 600 ml of distilled water, titrated with 0.1 N HC1 to a pH of 2, and held at a pH of 2 for 24 hours. The product, after being filtered, washed with 8 liters of distilled water, and air dried on the filter, had 95 ppm Na.

The resultant product (80 g) was treated for 24 hours with a solution of 80 g of octylamine in 160 g of DMSO, filtered, air dried and then held for subseouent treatments.

b) A 20 g. sample of the propped product of (a) above was reacted first with hydrogen sulfide in order to introduce $H_2S$ into the interlayer and then with a 100 g sample of titanium tetraisopropoxide. The titanium tetraisopropoxide reaction was conducted at room temperature for 3 days in a sealed polypropylene bottle, whereafter the resultant slurry was filtered, air-dried and calcined for 2 hours at 538°C (1000°F) in air. The product was a magadiite pillared with titanium disulfide.

Example 2

10.0 g of the layered metal dichalcogenide $TiS_2$ were reduced by contacting with a IM aqueous solution of $Na_2S_2O_4$. The reduced product and II.5 g of n-octylamine, 8.4g 37.1% HCl, and 100g $H_2O$ were placed in a Pyrex tube which was evacuated and sealed. The tube was heated to 100°C for 4 days, whereafter the contents of the tube were filtered and air dried. This product was stirred in $0_2$-free $H_20$ for 1 day, then filtered and dried under a flow of $N_2$. The solid thus obtained was then treated with tetraethylorthosilicate (5g TEOS/g solid) for 72 hours. After filtering and drying in $N_2$, the solid was calcined in $N_2$ at 500°C for 4 hours. The resulting silica-pillared $TiS_2$ exhibited increased n-hexane and water sorption and increased surface area over the $TiS_2$ starting material.

## Claims

1. A layered product comprising a layered chalcogenide of at least one element having an atomic number of 4, 5, 12 to 15, 20 to 33, 38 to 51, 56 to 83 and greater than 90, inclusive, and pillars of a chalcogenide of at least one element selected from Group IB, IIB, IIIA, IIIB, IVA, IVB, VA, VB, VIA, VIIA, VIIIA, of the Periodic Table of the Elements separating the chalcogenide layers, at least one of the chalcogens of the layered chalcogenide and said pillars being other than oxygen.

2. The product of claim 1 wherein said layered chalcogenide is non-swellable (as herein defined).

3. The product of claim 1 wherein said layered chalcogenide is a layered oxide and said pillars comprise at least one non-oxide chalcogenide.

4. The product of claim 3 wherein said layered oxide is a layered silicate, a layered titanate, a layered

oxide in which the layers have a perovskite-related structure or a layered metal oxide in which each layer has the general formula

$[M_x\square_y Z_{2-(x+y)}O_4]\alpha-$

wherein M is at least one metal of valence n where n is an integer between 0 and 7, □ represents a vacancy site, Z is a tetravalent metal, and wherein

$\alpha = 4y - x(n - 4)$

and $0 < x + y < 2$

5. The product of claim 3 wherein said pillars comprise a sulfide.

6. The product of claim 1 wherein said layered chalcogenide has the formula $MX_2$ where M is a transition metal and X is S, Se, or Te.

7. The product of claim 6 wherein the pillars are formed of an oxide.

8. The product of claim 7 wherein the pillars comprise polymeric silica.

9. A method for preparing the layered product of claim 1 comprising the steps of starting with a layered chalcogenide of at least one element having an atomic number of 4, 5, 12 to 15, 20 to 33, 38 to 51, 56 to 83 and greater than 90, inclusive, said layered chalcogenide having anionic sites associated therewith, physically separating the layers of the layered chalcogenide by introducing an organic cationic species between the layers at the anionic sites, introducing between the separated layers of the layered chalcogenide a compound capable of conversion to a chalcogenide and converting said compound to chalcogenide pillars separating adjacent layers of the layered chalcogenide, at least one of the chalcogens of the layered chalcogenide and the chalcogenide pillars being other than oxygen.

10. The method of claim 9 wherein said organic cationic species is an alkylammonium cation.

11. A catalyst composite comprising a layered product as claimed in claim 1 and a matrix material.

**Patentansprüche**

1. Schichtprodukt, das ein geschichtetes Chalkogen von mindestens einem Element mit der Atomzahl von 4, 5, 12 bis 15, 20 bis 33, 38 bis 51, 56 bis 83 und einschließlich größer als 90 aufweist, und Stützen eines Chalkogens von mindestens einem Element umfaßt, das aus der Gruppe IB, IIB, IIIA, IIIB, IVA, IVB, VA, VB, VIA, VIIA, VIIIA des Periodensystems der Elemente ausgewählt ist, die die Chalkogenschichten trennen, wobei mindestens eines der Chalkogene des geschichteten Chalkogens und der Stützen von Sauerstoff verschieden ist.

2. Produkt nach Anspruch 1, worin das geschichtete Chalkogen nicht quellbar ist (wie es hier definiert ist).

3. Produkt nach Anspruch 1, worin das geschichtete Chalkogen ein geschichtetes Oxid ist, und die Stützen zumindest ein chalkogen umfassen, das kein Oxid ist.

4. Produkt nach Anspruch 3, worin das geschichtete Oxid ein geschichtetes Silikat, geschichtetes Titanat, geschichtetes Oxid, in dem die Schichten eine dem Perovskit verwandte Struktur aufweisen, oder ein geschichtetes Metalloxid ist, worin jede Schicht die allgemeine Formel aufweist:

$[M_x\square_y Z_{2-(x+y)}O_4]\alpha-$

worin M zumindest ein Metall der Wertigkeit n ist, worin n eine ganze Zahl zwischen 0 und 7 ist, □ eine Leerstelle darstellt, Z ein vierwertiges Metall ist, und worin

$A = 4y - x(n - 4)$

und $0 < x + y < 2$.

5. Produkt nach Anspruch 3, worin die Stützen ein Sulfid umfassen.

6. Produkt nach Anspruch 1, worin das geschichtete Chalkogen die Formel $MX_2$ aufweist, worin M ein Übergangsmetall und X S, Se oder Te ist.

7. Produkt nach Anspruch 6, worin die Stützen aus einem Oxid gebildet sind.

8. Produkt nach Anspruch 7, worin die Stützen polymeres Siliziumdioxid umfassen.

9. Verfahren zur Herstellung des Schichtprodukts nach Anspruch 1, welches die Schritte umfaßt: Ausgang mit einem geschichteten Chalkogen von mindestens einem Element mit der Atomzahl von 4, 5, 12 bis 15, 20 bis 33, 38 bis 51, 56 bis 83 und einschließlich größer als 90, wobei das geschichtete Chalkogen damit verbundene anionische Plätze aufweist, physikalische Trennung der Schichten des geschichteten Chalkogens durch Einführung einer organischen kationischen Art zwischen die Schichten an den anionischen Plätzen, Einführung einer Verbindung zwischen die abgetrennten Schichten des geschichteten Chalkogens, die zur Umwandlung zu einem Chalkogen in der Lage ist, und Umwandlung der Verbindung in Chalkogen-Stützen, die die benachbarten Schichten des geschichteten Chalkogens trennen, wobei zumindest eines der Chalkogene des geschichteten Chalkogens und der Chalkogen-Stützen von Sauerstoff verschieden ist.

10. Verfahren nach Anspruch 9, worin die organische kationische Art ein Alkylammoniumkation ist.

11. Katalysatorverbundstoff, der ein geschichtetes Produkt nach Anspruch 1 und ein Matrixmaterial umfaßt.

**Revendications**

1. Un produit stratifié comprenant un chalcogénure en couche d'au moins un élément ayant un nombre atomique de 4, 5, 12 à 15, 20 à 33, 38 à 51, 56 à 83 et supérieur à 90, inclusivement, et des piliers d'un chalcogénure d'au moins un élément choisi dans les Groupes IB, IIB, IIIB, IVA, IVB, VA, VB, VIA, VIIA, VIIIA du Tableau Périodique des Eléments séparant les couches de chalcogénure, au moins l'un des chalcogénures du chalcogénure en couche et desdits piliers étant autre qu'un atome d'oxygène.

2. Le produit selon la revendication 1, dans lequel ledit chalcogénure est non gonflable (ainsi que défini ci-dessus).

3. Le produit selon la revendication 1, dans lequel ledit chalcogénure est un oxyde en couche et lesdits piliers comprennent au moins un chalcogénure non oxydique.

4. Le produit selon la revendication 3, dans lequel ledit oxyde est un silicate en couche, un titane en couche, un oxyde en couche, dont les couches ont une structure du type perovskite ou un oxyde métallique en couche dont chaque couche correspond à la formule générale:

$(M_x\square_y Z_{2-(x+y)}O_4)\alpha$

dans laquelle M est au moins un métal de valence n, n étant un nombre entier de 0 à 7, □ représente un site vacant, Z est un métal tétravalent et

$\alpha=4y-x(n-4)$ et

$0 < x+y < 2$

5. Le produit selon la revendication 3, dans lequel lesdits piliers comprennent un sulfure.

6. Le produit selon la revendication 1, dans lequel ledit chalcogénure en couche est de formule $MX_2$, où M est un métal de transition et X est S, Se ou Te.

7. Le produit selon la revendication 6, dans lequel les piliers sont formés d'un oxyde.

8. Le produit selon la revendication 7, dans lequel les piliers comprennent de la silice polymérisée.

9. Un procédé pour la préparation du produit stratifié selon la revendication 1, comprenant les étapes consistant à partir d'un chalcogénure en couche d'au moins un élément ayant un nombre atomique de 4, 5, 12 à 15, 20 à 33, 38 à 51, 56 à 83 et supérieur à 90 inclusivement, ledit chalcogénure en couche ayant des sites anioniques associés, à séparer les couches de chalcogénure en couche par introduction d'une espèce organique cationique entre les couches à l'endroit des sites anioniques, à introduire entre les couches séparées du chalcogénure en couche un composé convertible en chalcogénure et à convertir ledit composé en piliers de chalcogénure séparant les couches adjacentes du chalcogénure en couche, l'un au moins des chalcogènes du chalcogénure en couche et des noyaux de chalcogénures étant autre que l'oxygène.

10. Le procédé selon la revendication 9, dans lequel l'espèce organique est le cation alkylammonium.

11. Le catalyseur composite formé d'un produit en couche ainsi que revendiqué dans la revendication 1 et une matière formant support.